Europäisches Patentamt

European Patent Office

Office européen des brevets

(11) Veröffentlichungsnummer: **0 224 676**

**A1**

# (12) EUROPÄISCHE PATENTANMELDUNG

(21) Anmeldenummer: 86113705.7

(22) Anmeldetag: 03.10.86

(51) Int. Cl.⁴: **A61K 37/02** , C12N 15/00 , C12N 1/20 , //(C12N15/00,C12R1:44,1:19)

(30) Priorität: 08.10.85 DE 3535797

(43) Veröffentlichungstag der Anmeldung:
10.06.87 Patentblatt 87/24

(84) Benannte Vertragsstaaten:
AT BE CH DE FR GB IT LI LU NL SE

(71) Anmelder: **Bissendorf Peptide GmbH**
**Burgwedeler Strasse 25**
**D-3002 Wedemark 2(DE)**
Anmelder: **Gesellschaft für**
**Biotechnologische Forschung mbH (GBF)**
**Mascheroder Weg 1**
**D-3300 Braunschweig-Stöckheim(DE)**

(72) Erfinder: **Hesch, Rolf-Dieter, Prof. Dr.**
**Gartenweg 12**
**D-3004 Isernhagen 2(DE)**
Erfinder: **Collins, John, Dr.**
**Ägidienstrasse 5**
**D-3300 Braunschweig(DE)**
Erfinder: **Frank, Ronald, Dr. Dipl.-Chem.**
**Leibnizstrasse 8**
**D-3340 Wolfenbüttel(DE)**
Erfinder: **Maywald, Friedhelm, Dr. Dipl.-Biol.**
**Elzweg 32**
**D-3300 Braunschweig(DE)**
Erfinder: **Götz, Friedrich, Dr.**
**Pferseer Strasse 15**
**D-8900 Augsburg(DE)**
Erfinder: **Netzker, Roland, Dr. Dipl.-Biol.**
**Hoher Hof 30 c**
**D-3304 Wendeburg(DE)**
Erfinder: **Schwellnus, Konradt, Dr.**
**Dipl.-Chem.**
**Chemnitzstrasse 9**
**D-3300 Braunschweig(DE)**
Erfinder: **Meyerhans, Andreas**
**Haidkamp 27**
**D-2080 Pinneberg(DE)**
Erfinder: **Blöcker, Helmut, Dr.**
**Maschplatz 13**
**D-3300 Braunschweig(DE)**

(74) Vertreter: **Werner, Hans-Karsten, Dr. et al**
**Deichmannhaus am Hauptbahnhof**
**D-5000 Köln 1(DE)**

EP 0 224 676 A1

(54) **Arzneimittel enthaltend fermentationstechnisch hergestelltes Cardiodilatin und Verfahren zur Herstellung desselben.**

(57) Arzneimittel zur Behandlung des Bluthochdrucks, koronarer Herzerkrankungen, organischer und funktioneller vaskulärer Störungen, Gefäßerkrankungen bei erhöhter Katecholaminsekretion und zur Verbesserung der Arterien-, Venen und Kapillar-Durchblutung in allen Organgebieten enthaltend fermentationstechnisch hergestelltes CDL und ein Verfahren zur Herstellung des CDL sowie dessen Verwendung. Weiterhin betrifft die Anmeldung gentechnisch erzeugte Varianten von Staphylococcus carnosus und Escherichia coli für die Herstellung von Cardiodilatin sowie die Verwendung dieser Mikroorganismen zur Herstellung von CDL.

## Arzneimittel enthaltend fermentationstechnisch hergestelltes Cardiodilatin und Verfahren zur Herstellung desselben

Gegenstand der vorliegenden Erfindung ist ein Arzneimittel zur Behandlung des Bluthochdrucks, koronarer Herzerkrankungen, organischer und funktioneller vaskulärer Störungen, Gefäßerkrankungen bei erhöhter Katecholaminsekretion und zur Verbesserung der Arterien-, Venen und Kapillar-Durchblutung in allen Organgebieten sowie dessen Verwendung und Verfahren zur Herstellung dieses Arzneimittels. Weiterhin sind Gegenstand der vorliegenden Erfindung neue Mikroorganismen, deren Verwendung sowie Verfahren zu deren Erzeugung auf gentechnischen Wegen. Ferner ist Gegenstand der vorliegenden Erfindung ein neuer Vektor für die Herstellung dieser neuen Mikroorganismen sowie die Verwendung synthetischer Oligonukleotide zur Erzeugung des in die Mikroorganismen einzubauenden Gens.

Seit mehr als 15 Jahren wurde nach wirksamen Prinzipien im menschlichen Organismus gesucht, die die Natrium-und Wasserausscheidung beeinflußen; zusätzlich war immer unklar, wie das Herz einen Einfluß auf die Motilität des Gefäßsystems im Körper nimmt. Zahlreiche Befunde deuteten schon früh daraufhin, daß das Herz selbst in diese Regulation eingreifen kann, und es wurde lange nach nervalen Prinzipien gesucht, die aber letzlich nicht verifiziert werden konnten. Es wurden humorale Mediatoren vermutet; diese konnten aber nur hypothetisch dargestellt werden. Erst Mitte der 70iger Jahre konnte gefunden werden, daß im Herzen und zwar im Vorhof dieses Organs, Sekretgranula vorhanden sind, die Peptidhormonmediaotren absondern. Durch klassische Gewebsaufbereitung und chemische Extraktion konnte eine ganze Familie unterschiedlich langer Peptide dargestellt werden, die ganz unterschiedliche Wirkung auf die Natrium-und Wassersekretion des Körpers ausüben und zudem gefäßerweiternde Wirkung entfalten. Diese extraktiven Peptidgemische wiesen auch eine stark gefäßerweiternde Wirkung ohne einen Effekt auf die Natrium-und Wasserexkretionsfunktion der Niere auf. Erst die gentechnologischen Untersuchungstechniken erlaubten dann in einer überraschend kurzen Zeit in mehreren Laboratorien und unabhängig voneinander im Jahre 1984 die Extraktion der mRNA für ein Precursorprotein aus Rattenvorhöfen und Vorhöfen des menschlichen Herzens, wobei diese mRNA für Peptide mit vorbeschriebenen Eigenschaften codiert. In rascher Folge wurde Mitte 1984 die Struktur und die Nucleotidsequenz des Gens für Ratten-und humanes Precursorprotein von CDL beschrieben. Die Analyse des codierten Precursorproteins zeigte, daß das Gen für ein Signalpeptid codiert, gefolgt von der Aminosäuresequenz für CDL; dazwischen liegt wieder eine intervenierende Sequenz von 31 Aminosäuren mit einer N-und C-Terminalen Spaltstelle (Arg-Asp und Arg-Ser), die von dem natriuretischen Peptid (hANaP) gefolgt wird. Nach der Synthese wird das Precursorprotein enzymatisch in CDL und hANaP an den vorbeschriebenen Spaltstellen zerlegt, so daß jedes Peptid getrennt aus dem Precursorprotein von Herzvorhof in die Zirkulation abgegeben wird.

In den letzten Jahren sind atriale Extrakte aus Rattenvorhöfen des Herzens eingehend untersucht worden. Dabei wurde eine Reihe von verschiedenen Peptiden isoliert, die mehr oder weniger starke natriuretische und diuretische Wirkungen haben und die zusätzlich die glatte Muskulator von Gefäßen relaxieren können. Sowohl für die humanen Peptide als auch für die Peptide der Ratte konnte eine gemeinsamer Precursor aus klonierter cDNA dargestellt werden, welche aus atrialer mRNA präpariert wurde. Das humane Peptid had 151 Aminosäuren. Die ersten 25 Aminosäuren dieses Peptids entsprechen dem Peptidanteil, den man als Signalpeptid bezeichnet und welcher das Prozessieren entlang der ribosomalen Synthese und die anschließende Sekretion steuert. Danach wird ein Peptid mit 126 Aminosäuren, das gamma-hANaP freigesetzt, das exakt ein Molekulargewicht von 13.000 hat und in dieser Form in menschlichen Vorhöfen identifiziert wurde. Über die physiologische Wirkung von hANaP, ein stark diuretisches und natriuretsiches Peptid, wußte man aus tierexperimentellen Untersuchungen schon relativ viel, während Unterschungen am Menschen erst durch die Einführung des humanen hANaP Ende 1984 möglich wurde. So ist ein humanes atriales natriuretisches Peptid (hANaP) Gegenstand der deutschen Patentanmeldung P 34 43 257.4. Mit diesem Mittel steht ein neues wirksames Prinzip zur Therapie von Ödemen und Bluthochdruck durch Salzretention zur Verfügung.

Bluthochdruck, koronare Herzkrankheit, organische und funktionelle vaskuläre Störungen, Gefäßerkrankungen bei erhöhter Katecholaminsekretion und beeinträchtigte Arterien-, Venen und Kapillar-Durchblutung in allen Organgebieten sind Krankheiten, die heute in der westlichen Industriegesellschaft eine hohe Morbidität und Mortalität aufweisen. Noch immer haben präventive epidemiologische Maßnahmen nicht genügend gewirkt, wahrscheinlich auch aufgrund einer ungenügenden pathophysiologischen Kenntnis und eines unzureichenden Verständnisses der Ursache der Erkrankungen. Die Bereitstellung von CDL dürfte auch in der Diagnostik der Pathopysiologie dieser Erkrankungen weiterhelfen, nachdem entsprechende Bestimmungsmethoden hierfür aufgebaut worden sind. Weiterhin ist es möglich, auf therapeutischem Sektor die geschilderten Krankheitsbilder durch die Substitution mit einer natürlichen, körpereigenen

3

Substanz physiologisch zu beeinflußen. Der Nutzen solcher Therapie, die mit Sicherheit nebenwirkungsärmer ist als die mit gängigen, chemischen Pharmakotherapeutika, liegt im Bereich der heute verwendeten Calciumantanogisten. Die Entwicklung von CDL asl neuem Antihypertonikum, zur Therapie der koronaren Herzkrankheit und organischer sowie funktioneller arterieller Erkrankungen wird neue Verwendungsmöglichkeiten für synthetische Herzpeptide, die in der Natur vorkommen, eröffnen.

CDL besitzt im Gegensatz zu humanem atrialen natriuretischen Peptide (hANaP) keine natriuretische oder diuretische Wirkung. CDL ist daher selektiv zur Senkung eines hohen Blutdrucks, der durch Gefäßkontrakttion zustandekommt, geeignet. Ebenfalls läßt sich eine Relaxierung der koronaren Gefäße des Herzens erreichen. Durch den direkten vasodilatatorischen Effekt kann es zur Therapie funktioneller und organischer arterieller Verschlußkrankheiten sowie zur Verbesserung der arteriellen Durchblutung in allen Organgebieten herangezogen werden. Im Gegensatz zu bisher verwendeten Pharmazeutika handelt es sich aber bei CDL um eine physiologische Substanz des menschlichen Körpers, in diesem Fall aus endokrinen Organen des Herzens, und damit um einen natürlichen Modulator cardiovaskulärer Funktion. Damit wird ein natürliches humanes Peptid angeboten, mit dessen Hilfe sich defekte Körperfunktionen und Krankheiten durch die Substitution natürlicher, humaner Mediatorpeptide behandeln lassen.

CDL ist ein starkes vasorelaxierendes Peptid. Es entsteht in vivo in den Sekretgranula des Vorhofes des Herzens und wird von dort in das zirkulierende Blut abgegeben. CDL wird nach seiner Synthese enzymatisch im Zellinneren aus einem genetisch gesteuerten Vorläuferprotein herausgeschnitten. Dieser Prozeß setzt aber außerdem humanes artriales natriuretisches Peptid (hANaP) frei. Erfindungsgemäß ist es jedoch möglich, dem Körper CDL zuzuführen, ohne daß gleichzeitig hANaP entsteht. Humanes CDL hat ein Molekulargewicht von 7.368 und ist aus 67 Aminosäuren zusammengesetzt. Die klassische chemische Synthese von CDL ist angesichts der Länge des Moleküls von mehr als 45 Aminosäuren nicht möglich bzw. außerordentlich schwierig und nur um den Preis extremer und kostenträchtiger Reinigungsverfahren durchführbar. Mit der Produktion von längeren Peptiden wäre der erforderliche Reinheitsstandard nicht zu erreichen und eine therapeutische Anwendung am Menschen aus pharmokologischen und ethischen Gründen nicht zu vertreten. Andere Synthesewege eröffnen die Methoden der Enzymologie oder die rekombinante Gentechnolgie. Mit der enzymatischen Synthese langer Peptide wurden bisher jedoch noch nicht genügend Erfahrungen gemacht.

Aufgabe der vorliegenden Erfindung ist somit die gentechnologische Synthese von CDL und die Bereitstel lung .eines neuen Arzneimittels zur Behandlung des Bluthochdrucks (Hypertonie), koronarer Herzerkrankungen, organischer und funktioneller vaskulärer Störungen, Gefäßerkrankungen bei erhöhter Katecholaminsekretion und zur Verbeserung der Arterien-, Venen und Kapillar-Durchblutung in allen Organgebieten.

Diese Aufgabe wird durch ein Arzneimittel enthaltend fermentationstechnisch hergestelltes Cardiodilatin (CDL) in hochgereinigter Form gelöst. Die Herstellung von CDL erfolgt dadurch, daß eine gentechnisch erzeugte Variante von Staphylococcus carnosus oder Escherichia coli fermentiert und anschließend das CDL aus dem Kulturmedium isoliert wird.

Ferner ist Gegenstand der vorliegenden Erfindung die Verwendung von mikrobiologisch synthetisierten CDL in hochgereinigter, injizierbarer Form zur Behandlung des Bluthochdrucks, koronarer Herzerkrankungen, organischer und funktioneller vaskulärer Störungen, Gefäßerkrankungen bei erhöhter Katecholaminsekretion und zur Verbesserung der Arterien-, Venen und Kapillar-Durchblutung in allen Organgebieten. Außerdem betrifft die vorliegenden Erfindung die Verwendung von CDL zur Herstellung von Arzneimitteln zur Behandlung des Bluthochdrucks, koronarer Herzerkrankungen, organischer und funktioneller vaskulärer Störungen, Gefäßerkrankungen bei erhöhter Katecholaminsekretion und zur Verbesserung der Arterien-, Venen und Kapillar-Durchblutung in allen Organgebieten.

Weiterer Gegenstand der vorliegenden Erfindung sind neue gentechnisch erzeugte Varianten von Staphylococcus carnosus und Escherichia coli sowie deren Verwendung zur Herstellung von CDL. Aufgabe der vorliegenden Erfindung ist es auch, ein Verfahren zur Erzeugung einer Variante von Staphylococcus carnosus oder Escherichia coli für die Herstellung von CDL zur Verfügung zu stellen. Diese Aufgabe wird dadurch gelöst, daß vollsynthetische DNA in einen Vektor eingebaut und anschließend als Hybrid-Plasmid in Staphylococcus carnosus oder Escherichia coli eingeschleust wird. Gegenstand der vorliegenden Erfindung ist demgemäß derner ein Vektor enthaltend vollsynthetisch hergestellte DNA mit einer Sequenz für die Produktion von CDL.

Die mRNA-Sequenz für das CDL-hANaP-Vorläuferprotein wurde kürzlich von Nakayame und Mitarbeitern publiziert (Nature 310, 23. August 1984, S. 699 ff.) Die Kenntnis der mRNA-Sequenz erlaubt die vollsynthetische Herstellung des isolierten Gens (196 Basenpaare + Tails) nun für CDL auf gensynthetischem Wege. Hierzu wird das synthetisches Gen in einen Vektor eingebaut, der anschließend als Hybrid-Plasmid in einen Mikroorganismus inkorporiert wrid; von diesen Mikroorganismus wird CDL dann produziert und aus dem Kulturmedium hochgereinigt. Das gewonnene hochreine Peptid (Reinheit mehr als 98%) wird ampulliert und auf biologische und toxikologische Wirkung untersucht. Das gewonnene Produkt weist Pyrogenfreiheit und Freiheit von Verunreinigungen durch die gentechnologische Herstellung auf.

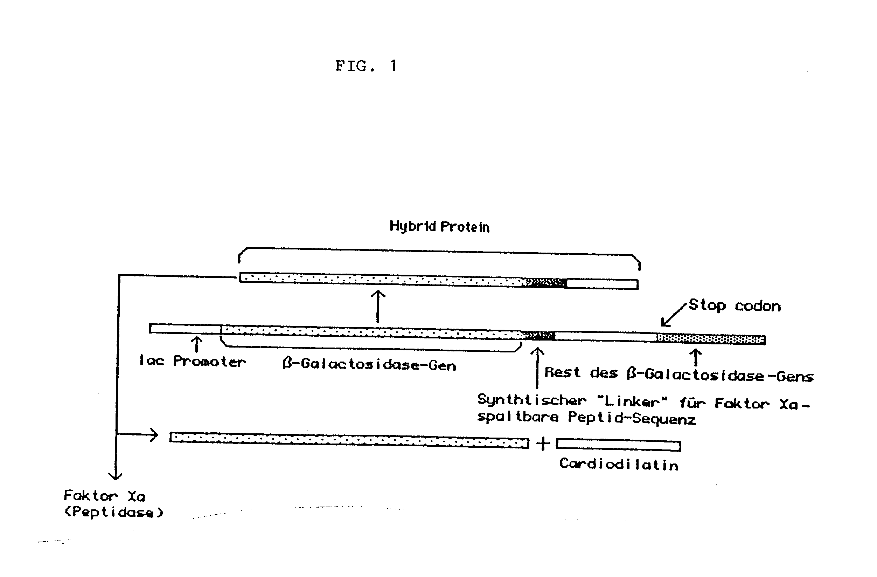

Die Herstellung von CDL auf gentechnischem Wege kann insbesondere mit Escherichia coli oder Staphylococcus carnosus durchgeführt werden. Hierbei kann man das Peptid an ein größeres, möglichst hydrophobes Protein (z.B. $\beta$-Galaktosidase) anhängen, so daß das unlös liche Präzipitat (Fusionsprotein) anschließend über die Affinitäts-Chromatographie (für den $\beta$-Galaktosidase-Anteil) gereinigt und abgespalten werden kann. In Fig. 1 ist der Einbau des Gens in den Vektor dargestellt.

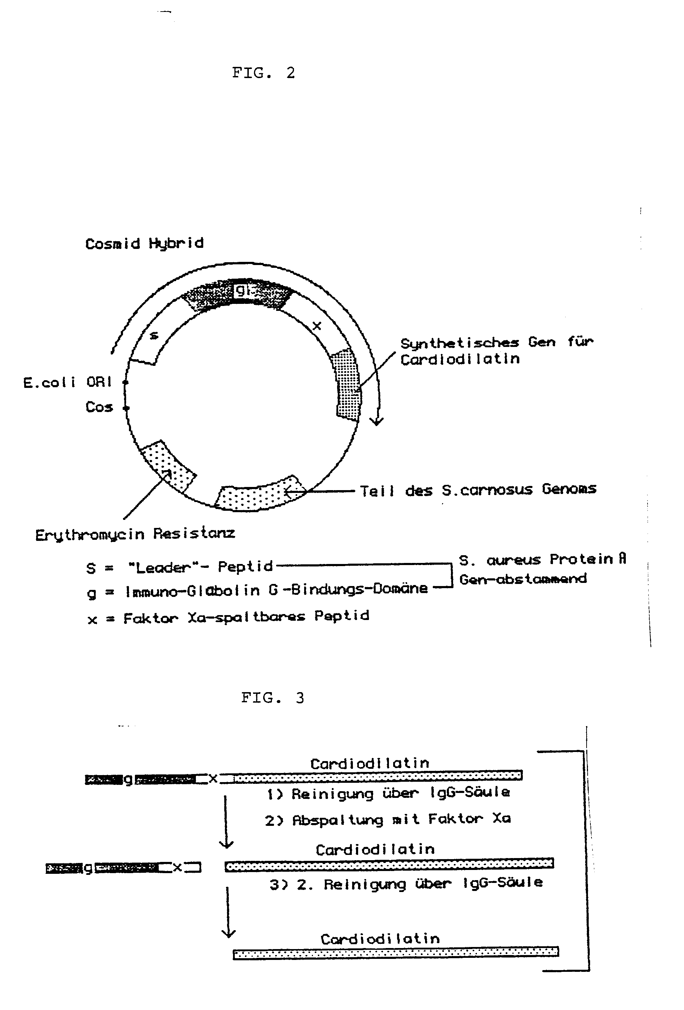

Staphylococcus carnosus hat als Wirtsstamm den Vorteil, daß der Organismus in Nahrungsmitteln - schon vorhanden ist (z.B. Trockenwurst) und in der Lage ist, Proteine ins Medium auszuschleusen. Das gewünschte CDL-Peptid wird als Fusions-Peptid produziert und gereinigt, anschließend durch proteolytische Spaltung freigesetzt und weiter gereinigt. Das Prinzip der Hybrid-Plasmid-Gewinnung ist in Fig. 2 dargestellt.

Dieses Hybrid-Plasmid wird in Staphylococcus carnosus eintransformiert, wo es im Chromosom integriert wird. Das Hybrid-Plasmid im Medium ist in Fig. 3 dargestellt.

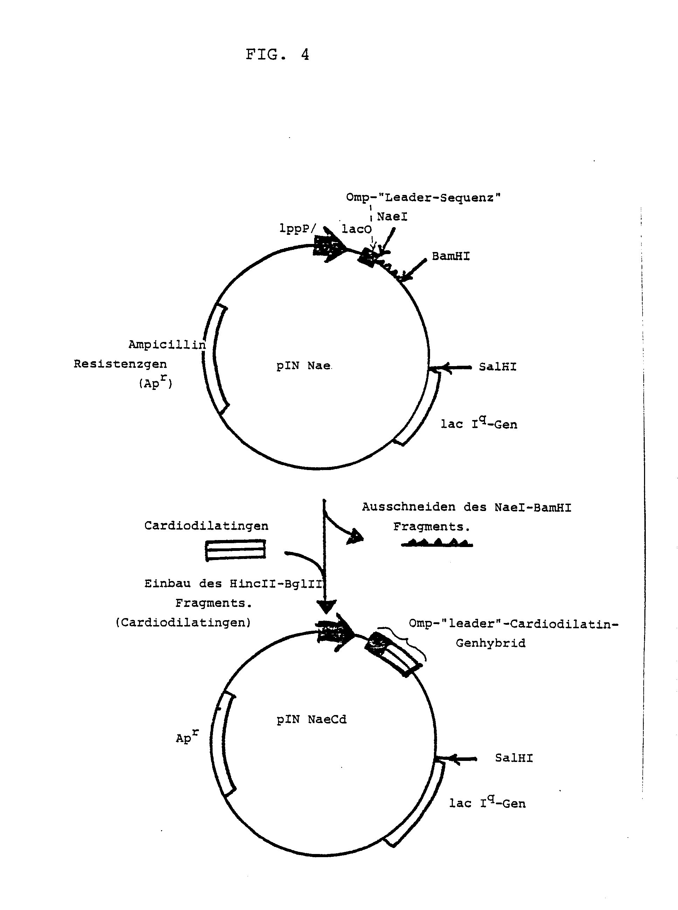

Eine weitere Ausführungsform der Erfindung verwendet die Expression von Cardiodilatin aus E. coli mittels eines Ausschleusungsvektors. Als Ausgangssystem dient der von Inouye konstruierte pINIII ompA-Vektor (Ghrayeb et al.: EMBO J. 3, 2437-2442, 1984), der zum pIN Nae (E.coli DH1 pIN III omp-Nae-Cd, NCIB 12323) umgebaut wurde (Fig. 4). Dieses Plasmid trägt zur Selektion ein Ampicillin-Resistenzgen (Ap ') und kodiert die "Leadersequenz" des äußeren Membranproteins ompA.

Die Transkription dieses Peptids wird durch den Lipoprotein-Promoter 1ppP und den Promoter-Operator des Lactose-Operons LacO reguliert. Dadurch kann die Transkription durch den Repressor LacI blockiert werden. Dieser Repressor wird ebenfalls vom Plasmid kodiert (LacI$^q$ -Gen).

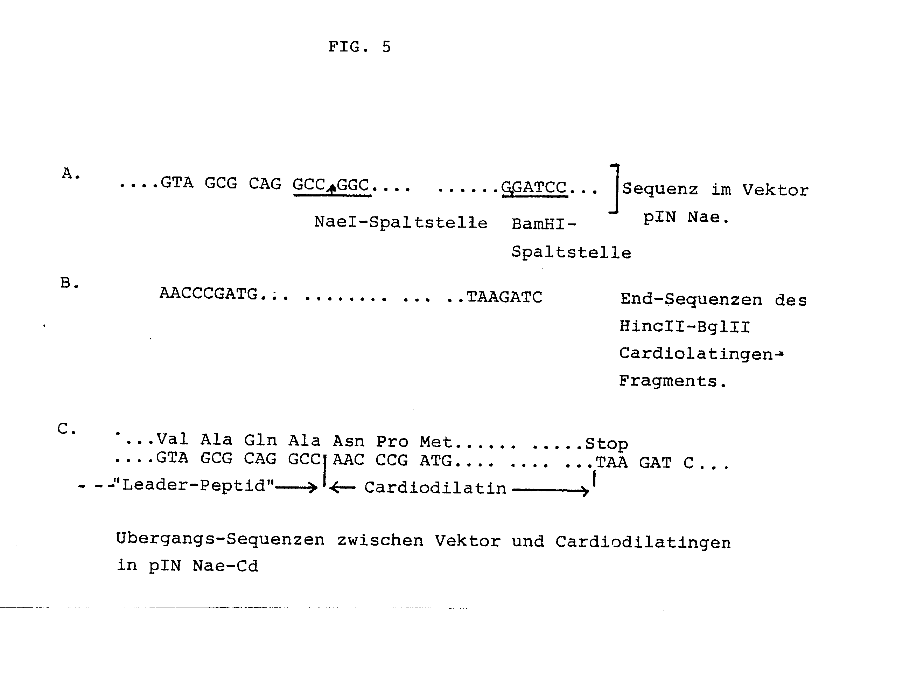

Eine Induktion erfolgt beispielsweise durch Zugabe von IPTG (Isopropylthiogalaktosid). Am Ende der "Leadersequenz" befindet sich eine Schnittstelle der Restriktionsendonuklease NaeI (Kessler et al.: Gene 33, 1-102, 1985), wodurch es ermöglicht wird, direkt hinter dem "Leader" ein beliebiges Genfragment mit glatten Enden unter Einhaltung des Leserahmens einzusetzen. Zwischen dieser NaeI und der folgenden BamHI-Schnittstelle wurde das synthetische Cardiodilatin-Gen als HincII-II-Fragment aus pGV 451 Cd - (hinterlegt bei der DSM unter der Hinterlegungs-Nr. 3527 und bei der NCIMB unter der Hinterlegungs-Nr. 12167) eingesetzt (Fig. 4). Dies ist möglich, da HincII glatte Enden hervorbringt und BglII zu BamHI kompatible überstehende Enden erzeugt. Auf diese Weise entsteht als primäres Translationsprodukt ein Fusionspeptid aus der "Leadersequenz" und Cardiodilatin (Sequenz der Übergangsstelle; Fig. 5). Die "Leadersequenz" dient als Signal zum Transport durch die innere Zellmembran und wird bei diesem Vorgang abgespalten.

Das nach der Transformation des pIN Nae-Cd Plasmids in E.coli DH1 pIN III omp-Cd (NCIB 12322) gebildete Peptid wurde durch Azeton-Fällung oder Säurefällung aus dem Medium konzentriert und gereinigt. Die Testung ergab gefäß-relaxierende Wirkung.

Als Alternative zur Expression in E.coli wurde ein Sekretionsvektor für Staphylococcus carnosus entwickelt, wie in den Beispielen näher erläutert wird.


Beispiel


1. Herstellung des synthetischen Cardiodilatin-Gens

28 Oligonucleotide (Numerierung wie in der Abbil dung) wurden durch Festphasensynthese auf segmentierten Cellulosepapierträger (Frank, R. et al. Nucl. Acids Res. 11, 4365-4377, 1983) hergestellt. Die einzelnen Oligonucleotide wurden nach Blöcker, H. und Frank, R., in: S. Silver (ed.) Biotechnology Potentials and Limitations, Dahlem Workshop Rep. LS 35, Springer-Verlag (in press) gereinigt.

Die Fragmente wurden in einer Einstufen-Eintopf-Reaktion zum Doppelstrang ligiert und in das HincII-BglII gespaltene pGV451-Plasmid eingebaut. Mit dieser DNA wurde E.coli JM83 transfomiert. Das Cardiodilatin-Gen wurde mit der Methode von Volckaert, G. et al. (1984) Gene Analysis Techniques 1, 52-59 sequenziert.


2. Herstellung des Staphylococcus carnosus-Stammes mit der Cardiodilatin-Lipase-Genfusion

## Tabelle

### Angewandte Stämme

| | |
|---|---|
| E.coli DH1 | (DSM 3523) und (NCIB 12163) |
| E.coli JM83 pGV451 | (DSM 3526) und (NCIB 12166) |
| E.coli 5K pBR325 | (Kommerziell erhältlich: |
| | Bethesda Research Laboratories |
| | Inc.) |
| S. carnosus TM300 | (DSM 3528) und (NCIB 12168) |
| S. carnosus pLip PS1 | (DSM 3529) und (NCIB 12169) |
| E.coli pBRX | (DSM 3524) und (NCIB 12164) |
| E.coli pBRXCd | (DSM 3525) und (NCIB 12165) |
| S. carnosus pLipXCd | (DSM 3536) |
| E.coli JM83 pGV451-Cd | (DSM 3527) und (NCIB 12167) |
| E.coli DH1 pIN III omp-Nae-Cd | (NCIB 12323) |
| E.coli DH1 pIN III omp-Cd | (NCIB 12322) |

### Methoden

Isolierung von Plasmid DNA, Analyse von Plasmid DNA durch Restriktionsenzymspaltung und Trennung der Bruchstücke durch Agarose-Gel-Elektrophorese, Ligation verschiedener DNA-Fragmente miteinander, Isolierung von DNA-Fragmenten aus Gel-Schnitten und Transformation in E.coli sind nach Protokollen, die in "Molecular Cloning, a Laboratory Manual" (Maniatis, T. et al. (1982) Cold Sp;ring Harbor Lab., New York) beschrieben sind, durchgeführt worden. Die benutzten Restriktionsendonukleasen wurden von Bethesda Research Laboratories (BRL) oder Boehringer Mannheim bezogen. Dasselbe gilt für T4-DNA-Ligase, DNA-Polymerase (Klenow-Fragment) und alkalische Phosphatase (aus Kälberdem). Trypton, Pepton und Hefeextrakt wurden von Difco, Antibiotika von Sigma und alle anderen Chemikalien von Merck oder Sigma gekauft.

Die benutzten oder konstruierten Bakterienstämme wurden bei der Deutschen Sammlung von Mikroorganismen (DSM) und den National Collections of Industrial and Marine Bacteria (NCIB) hinterlegt.

In der Tabelle sind die Hinterlegungsnummern zusammengefaßt.

Transformation in S. carnosus ist nach Götz et al. (1983) Mol. Gen. Genetic 189, 340-342, durchgeführt worden.

Diese Arbeitsgruppe hat die Grundlage für die hier beschriebene Verfahrensweise erarbeitet. Die Konstruktion des Plasmids pLipS1 und die Expression von Lipase-β-Lactamase-Fusionsproteinen in Staphylococcus carnosus sind beschrieben. Das Plasmid pLipPS1 vermittelt Chloramphemicolresistenz und trägt ein Lipasegen aus Staphylococus hyicus. Die Lipase wird exprimiert und in das Medium ausgeschieden. Dasselbe gilt für Lipase-β-Lactamase-Fusionsproteine in Abhängigkeit von der Lage der Fusionsstellen.

Diese Verfahrensweise wird auch für die Cardiodilatinproduktion genutzt. Ein Problem war dabei die Abspaltung des nativen Cardiodilatins vom Lipase-CDL-Fusionsprotein. Eine Möglichkeit zur spezifischen Spaltung von Proteinen wurden von Nagai und Thögersen, Nature 309, 810-812 (1984), beschrieben. Dieses Autoren benutzten zu diesem Zweck den Blutgerinnungsfaktor Xa, der Proteine spezifisch hinter der Erkennungssequenz I1e-Glu-Gly-Arg schneidet. Daher wurde zunächst ein Linker synthetisiert, der die Xa-Erkennungssequenz kodiert und folgende Struktur besitzt:

Stul Bg1ll
EcoRI AATTCATCGAGGTAGGCCTAGATCTG
GTAGCTCCCATCCGGATCTAGACTTAA EcoRI
lle Glu Gly Arg

Dieser Linker wurde so konzipiert, daß sich die Kodons für lle-Glu-Gly-Arg direkt an den Leserahmen des Lipasegens anfügen, wenn dieser in die EcoRI-Schnittstelle dieses Gens inseriert wird. Eine Stul-Schnittstelle wurde so gelegt, daß sich synthetische Gene mit glatten 5'Enden in einer Weise ligieren lassen, daß das erste Kodon des Gens an die Xa-Erkennungssequenz anschließt.

Um die Lipase-Cardiodilatin-Genfusion vorzubereiten, sind folgende Schritte durchzuführen:

Im EcoRI site von pBR325 ist der "Linker" Oligonucleotid

LX = $^5$'AATTCATCGAGGGTAGGCCTAGATCTGA$^3$'
CTAGCTCCCATCCGGATCTAGACTTAA$_5$'
Stul BglII

eingebaut. Das Plasmid mit Linker wurde pBRX genannt. Aus pBRX sind zwei Fragmente isoliert worden - (Stul-SalHI-A-Fragment, Bglll-SalHI-B-Fragment), die dann mit dem Hincll-Bglll-Cardiodilatin-Gen-Fragment von pGV451-Cd ligiert wurden und in E.coli 5K transformiert worden sind. Das daraus entstehende Plasmid heißt pBRXCd. Das kleinste EcoRI-Fragment (oder EcoRI-Bglll-Fragment) dieses Plasmids wurde in pLip - (von S. carnosus pLip) in die EcoRI-Stelle (oder in EcoRI-Sau3A gespaltene pLipPS1) im Lipase-Gen einligiert und S. carnosus TM300 eintransformiert. Der daraus resultierende Stamm heißt S. carnosus pLipXCd und stellt einen Produktionsstamm für ein Lipase-Cardiodilatin-Fusionspeptid dar. Um die richtigen Leseraster zu erhalten und die spätere proteolytische Abspaltung des Cardiodilatins zu ermöglichen, ist der Linker X eingebaut worden.

Die Sequenz an der Übergangsstelle ist folgende:

Lipase Gen*          Vom Linker X      Anfang des Cardiodilatin-
                                       Gens

——TTT,GGT,ATG,GAA,TTC,ATC,GAG,GGT,AGG,AAC,CCG,ATG,TAC,AAC———
         EcoRI—Spaltstelle


Leu-Gly-Met-Glu-Phe-Ile-Glu-Gly-Arg-Asn-Pro-Met-Tyr-Asn———

Aminosäure-          Erkennungs-       Anfang des Cardiodilatin-
Sequenz des          sequenz für       Peptids
Fusionspeptids       Faktor Xa
                     Protease


                                       Spaltstelle
                                       für Faktor Xa


*: Sequenz nach Götz, F. et al. (1985) Nucl. Acids Res.
   13, 5895-5906


Das so erhaltene Cardiodilatin wird in reiner Form erhalten. Insbesondere ist es frei von Verunreinigungen mit hANaP. Cardiodilatin wird in einer für die Injektion geeigneten Dosis verabreicht. Diese Dosis enthält eine effektive Menge an Cardiodilatin, welches

) frei von Verunreinigugnen mit anderen Peptiden, insbesondere hANaP, ist und

b) eine Trägerlösung, bestehend aus einem physiologisch verträglichen Lösungsmittel für Cardiodilatin, enthält. Die Gesamtmenge einer Verabreichung von Cardiodilatin in einer einmaligen Einheitsdosis beträgt typischerweise zwischen 50 bis 500 μg, vorzugsweise 100 bis 300 μg.

Eine typische Einheitsdosis beläuft sich auf 50 bis 500 μg, vorzugsweise 100 bis 300 μg.

Als Cardiodilatin-Trägerlösung kann eine für die cardiovaskuläre Therapie übliche Trägerlösung verwendet werden, die physiologische Kochsalzlösung, die ggf. Hilfsstoffe wie Manitol oder ein Trägerprotein, z.B. humanes Serumalbumin enthalten kann.

Die Konzentration der Lösung an Cardiodilatin beträgt typischerweise 50 bis 500 μg/ml. Cardiodilatin wird in einer Dauerinfusion mit 0,1 bis 5 μg/kg/min. verabreicht.

Weitere geeignete Applikationsformen von Cardiodilatin sind transdermale, örtliche und buccale Anwendungen. Darüber hinaus ist es möglich, Cardiodilatin in Mini-oder Mikrokapseln subkutan zu applizieren, um eine langsame Freisetzung der Inhaltsstoffe zu erreichen. Weiterhin kann es vorteilhaft sein, Cardiodilatin in verkapselter Form in geeigneten Kapseln zu verabreichen, um in Magen-Darm-Trakt eine verzögerte Freisetzung der Inhaltsstoffe zu gewährleisten.

**Ansprüche**

1. Arzneimittel zur Behandlung des Bluthochdrucks und koronarer Herzerkrankungen, organischer und funktioneller vaskulärer Störungen, Gefäßerkrankungen bei erhöhter Katecholaminsekretion und zur Verbesserung der Arterien-, Venen und Kapillar-Durchblutung in allen Organgebieten enthaltend fermentationstechnisch hergestelltes Cardiodilatin (CDL) in hochgereinigter Form.

2. Verfahren zur Herstellung von CDL, dadurch gekennzeichnet, daß eine gentechnisch erzeugte und zur Expression von CDL befähigte Variante von Staphylococcus carnosus oder Escherichia coli fermentiert und anschließend das CDL aus dem Kulturmedium isoliert wird.

3. Verfahren zur Herstellung von CDL, dadurch gekennzeichnet, daß Staphylococcus carnosus DSM 3536 oder Escherichia coli DSM 3527 fermentiert und anschließend das CDL aus dem Kulturmedium isoliert wird.

4. Verwendung von mikrobiologisch synthetisierten CDL in hochgereinigter, injizierbarer Form zur Behandlung des Bluthochdrucks, koronarer Herzerkrankungen, organischer und funktioneller vaskulärer Störungen, Gefäßerkrankungen bei erhöhter Katecholaminsekretion und zur Verbesserung der Arterien-, Venen und Kapillar-Durchblutung in allen Organgebieten.

5. Verwendung von CDL zur Herstellung von Arzneimitteln zur Behandlung des Bluthochdruckes, koronarer Herzerkrankungen, organischer und funktioneller vaskulärer Störungen, Gefäßerkrankungen bei erhöhter Katecholaminsekretion und zur Verbesserung der Arterien-, Venen und Kapillar-Durchblutung in allen Organgebieten.

6. Staphylococcus carnosus DSM 3536

7. Verwendung von Staphylococcus carnosus nach Anspruch 6 zur Herstellung von CDL.

8. Escherichia coli DSM 3527

9. Verwendung von Escherichia coli nach Anspruch 8 zur Herstellung von CDL.

10. Vektor enthalten in DSM 3536 und DSM 3527 enthaltend vollsynthetisch hergestellte DAN mit einer Sequenz für die Produktion von CDL.

11. Verfahren zur gentechnischen Erzeugung einer Variante von Staphylococcus carnosus oder Escherichia coli für die Herstellung von CDL, dadurch gekennzeichnet, daß vollsynthetische DNA in einen Vektor eingebaut und anschließend als Hybrid-Plasmid in Staphylococcus carnosus oder Escherichia coli eingeschleust wird.

FIG. 1

Hybrid Protein

Stop codon

lac Promoter  ß-Galactosidase-Gen

Rest des ß-Galactosidase-Gens

Synthtischer "Linker" für Faktor Xaspaltbare Peptid-Sequenz

+ Cardiodilatin

Faktor Xa
(Peptidase)

0 224 676

FIG. 2

Cosmid Hybrid

Synthetisches Gen für
Cardiodilatin

E.coli ORI

Cos

Teil des S.carnosus Genoms

Erythromycin Resistanz

S = "Leader"- Peptid ————————————— S. aureus Protein A
                                                   Gen-abstammend
g = Immuno-Glabolin G -Bindungs-Domäne ——

x = Faktor Xa-spaltbares Peptid

FIG. 3

Cardiodilatin

1) Reinigung über IgG-Säule

2) Abspaltung mit Faktor Xa

Cardiodilatin

3) 2. Reinigung über IgG-Säule

Cardiodilatin

FIG. 4

Omp-"Leader-Sequenz"
NaeI

lppP/   lacO

BamHI

Ampicillin
Resistenzgen
(Ap$^r$)

pIN Nae.

SalHI

lac I$^q$-Gen

Ausschneiden des NaeI-BamHI
Fragments.

Cardiodilatingen

Einbau des HincII-BglII
Fragments.
(Cardiodilatingen)

Omp-"leader"-Cardiodilatin-
Genhybrid

Ap$^r$

pIN NaeCd

SalHI

lac I$^q$-Gen

## FIG. 5

A.

    ....GTA GCG CAG <u>GCC<sub>▲</sub>GGC</u>.... ......<u>GGATCC</u>... ] Sequenz im Vektor

                NaeI-Spaltstelle  BamHI-       pIN Nae.

                                      Spaltstelle

B.

      AACCCGATG... ........ ... ..TAAGATC     End-Sequenzen des

                                          HincII-BglII

                                          Cardiolatingen-

                                        Fragments.

C.

      ·...Val Ala Gln Ala Asn Pro Met...... .....Stop

      ....GTA GCG CAG GCC|AAC CCG ATG.... .... ...TAA GAT C...

  - --"Leader-Peptid"——→|←— Cardiodilatin ——————→|

Ubergangs-Sequenzen zwischen Vektor und Cardiodilatingen
in pIN Nae-Cd

0 224 676

EUROPÄISCHER TEILRECHERCHENBERICHT,
der nach Regel 45 des Europäischen Patentübereinkommens für das weitere Verfahren als
europäischer Recherchenbericht gilt

Europäisches
Patentamt

Nummer der Anmeldung

## EINSCHLÄGIGE DOKUMENTE

EP 86113705.7

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| P,X | EP - A2 - O 159 943 (MITSUBISHI CHEMICAL INDUSTRIES LIMITED) <br><br> * Ansprüche 1,10-13; Seite 1 * <br><br> ---- | 1,2,5, 11 | A 61 K 37/02 <br> C 12 N 15/00 <br> C 12 N 1/20 <br> // (C 12 N 15/00 <br> C 12 R 1:44 <br> C 12 R 1:19) |

### RECHERCHIERTE SACHGEBIETE (Int. Cl.4)

A 61 K 37/00
C 12 N 15/00
C 12 N 1/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-3,5-11

Unvollständig recherchierte Patentansprüche: —

Nicht recherchierte Patentansprüche: 4

Grund für die Beschränkung der Recherche:

(Verfahren zur therapeutischen Behandlung des menschlichen oder tierischen Körpers, Art. 52(4) EPÜ)

| Recherchenort | Abschlußdatum der Recherche | Prüfer |
|---|---|---|
| WIEN | 06-03-1987 | STÖCKLMAYER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein- stimmendes Dokument

EPA Form 1505.1   08.82